(19)

Europäisches
Patentamt
European
Patent Office
Office européen
des brevets

(11) **EP 4 224 479 A1**

(12) **EUROPEAN PATENT APPLICATION**

(43) Date of publication:
**09.08.2023 Bulletin 2023/32**

(21) Application number: **23153706.9**

(22) Date of filing: **27.01.2023**

(51) International Patent Classification (IPC):
**G16B 40/00** (2019.01)   **G16H 50/30** (2018.01)

(52) Cooperative Patent Classification (CPC):
**G16B 40/00; G16H 50/30;** G16H 30/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.02.2022 TW 111104581**

(71) Applicants:
 • **National Cheng Kung University Hospital
   704 Tainan City (TW)**
 • **National Cheng Kung University
   Tainan City 701 (TW)**

(72) Inventors:
 • **Shen, Meng-Ru
   701 Tainan City (TW)**

 • **Tsai, Yi-Shan
   701 Tainan City (TW)**
 • **Li, Chung-I
   701 Tainan City (TW)**
 • **LIN, Peng-Chan
   701 Tainan City (TW)**
 • **Su, Pei-Fang
   701 Tainan City (TW)**
 • **Liao, Yi-Huan
   701 Tainan City (TW)**
 • **Lai, Yu-Husan
   701 Tainan City (TW)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **METHOD FOR PREDICTING CANCER PROGNOSIS AND MODEL THEREFOR**

(57) A method for predicting cancer prognosis is disclosed, and the method comprises capturing a reference radiomics and obtaining reference pathological eigenvalues, wherein the reference pathological eigenvalues are based on pathological features of a reference patients, and the pathological features comprise genomic features, gene expression, test values or a combination of two or more thereof. Then, capturing a test radiomics and obtaining test pathological eigenvalues are performed, wherein the test pathological eigenvalues are based on pathological features of a test patients, and the pathological features comprise genomic features, gene expression, test values or a combination of two or more thereof. A mathematical formula is used to calculate a prognostic index based on the aforementioned reference radiomics, reference pathological eigenvalues, test radiomics and test pathological eigenvalues, and the prognostic change risk of the test patient is evaluated according to the prognostic index.

FIG. 1

EP 4 224 479 A1

**Description**

**FIELD OF THE INVENTION**

The invention relates to a method for predicting changes in the prognosis of cancer patients, and particularly to a method for predicting the recurrence of cancer prognosis.

**BACKGROUND OF THE INVENTION**

Colorectal cancer is one of the most common malignant tumors in the world, and its mortality rate ranks third among the top ten cancers in the United States. With the westernization of diet, the number of people suffering from colorectal cancer in Taiwan has gradually increased to rank first and second in cancer mortality rates for Taiwanese men and women respectively; several studies have shown that the recurrence rate of colorectal cancer is as high as 40% within 2 years after treatment, and the recurrence rate is still 5% in the 5th year after treatment; in recent years, early postoperative recurrence of colorectal cancer has been evaluated by clinical features or serum markers. Among them, the mechanisms of inflammation and angiogenesis may be the reasons for the recurrence of colorectal cancer, and the molecular markers of circulating tumor cells (CTC) can be used as prognostic factors for the recurrence risk of colorectal cancer. Genes in human feces are associated with colorectal cancer, and some genes have molecular significance in cancer biology or molecular medicine. Studies have shown that the growth arrest specific 2 (GAS2) is overexpressed in the stool of patients with recurrent colorectal cancer, and it has been shown to be a target gene for chemotherapy. In addition, placenta-specific gene 8 (PLAC8) has been reported to be involved in Epithelial-mesenchymal transition (EMT) in colorectal cancer. However, at present, the medical community seems to have a partial understanding of the molecular mechanism of colorectal cancer recurrence, but so far there is still no effective and accurate prediction or method for colorectal cancer recurrence.

Breast cancer is the most commonly diagnosed malignant disease in women, and ranks first in the global female cancer mortality rate. And 45% of patients are in recurrence after treatment. It reaches 13% in the first to second years after breast cancer diagnosis, and then gradually decreases. About 50% of recurrence cases occur in the first 5 years after surgery, but there is an average annual recurrence risk rate of 4.7% in 5 to 8 years after surgery, and a 3.4% recurrence risk rate in 8 to 12 years after surgery. Furthermore, the most common sites of breast cancer metastases are the lungs, liver and bones, and metastases to the bones are usually associated with severe pain, bone loss and increased risk of bone fracture; in addition, menopause is also one of the most important causes of osteoporosis in women. Breast cancer patients may enter menopause early due to treatment, and their bone loss rate may be faster than that of women with natural menopause, leading to an increased risk of osteoporosis. In combination with the anti-estrogen therapy used in the treatment of breast cancer, it also accelerates bone loss, so breast cancer patients are often accompanied by osteoporosis.

From the foregoing content, it can be easily understood that the tracking and prediction of the prognosis of cancer is a very important part of the current medical profession. If the prognostic risk of the patient can be predicted through the existing pathological features and clinical data after treatment, such as cancer recurrence, this plays a role in predicting and preparing in advance for patient's prognostic care and the health quality of the patient's prognosis is also more guaranteed. Therefore, after deliberate research and experimentation with perseverance and resilience, the instant applicant overcomes the deficiencies in the prior art and conceives inventive ideas of the present invention, and the following is a brief description of the present invention.

**SUMMARY OF THE INVENTION**

In order to solve the above-mentioned problems, an object of the present invention is to provide a method for predicting cancer prognosis, which can simultaneously use high-dimensional tensor data and covariate to perform discriminant analysis, the method is preferably performed by a computer. Furthermore, the case where the covariance matrix of covariate under different types are different is comprised, and the CATCH/CATCH+ model established based on the simultaneous correction of the linear relationship between tensor and covariate is used to discriminate the types. Among them, CATCH+ model can be applied to classification and discrimination of more than two types. The method comprises: capturing a reference radiomics, wherein the reference radiomics is based on a reference image, and the reference image is a lesion medical image of a reference patient; obtaining a reference pathological eigenvalue, wherein the reference pathological eigenvalue is based on pathological features of the reference patient, and the pathological features comprise genomic features, gene expression, test values or a combination of two or more thereof; capturing a test radiomics, wherein the test radiomics is based on a test image and the test image is a lesion medical image of a test patient; obtaining a test pathological eigenvalue, wherein the test pathological eigenvalue is based on pathological features of the test patient, and the pathological features comprise genomic features, gene expression, test values or a combination of two or more thereof; and using a mathematical formula to calculate a prognostic index, wherein a risk level of prognostic change of the test patient is evaluated according to the prognostic index, and the mathematical formula is as follows:

$$prognostic\ index = \frac{f_2(U_2, X_2)\pi_2}{f_1(U_1, X_1)\pi_1}$$

, wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

Preferably, the step of the capturing the reference radiomics comprises: capturing a reference lesion image, capturing a plurality of image feature variable values from the reference lesion image, outputting a reference image format data, and then normalizing the reference image format data with a dimensionality reduction matrix to obtain the reference radiomics, wherein the reference lesion image is a lesion image of the reference patient; and the step of the capturing the test radiomics comprises: capturing a test lesion image, capturing a plurality of image feature variable values from the test lesion image, outputting a test image format data, and then normalizing the test image format data with a dimensionality reduction matrix to obtain the test radiomics, wherein the test lesion image is a lesion image of the test patient.

Preferably, the gene expression comprises RNA sequencing expression or protein expression.

Preferably, the genomic features comprise gene copy number, gene mutant site or single nucleotide polymorphisms (SNPs).

Preferably, when the gene expression is an RNA sequencing, the method further comprises: normalizing a gene reading with a following formula to obtain the RNA sequencing expression,

$$RNA\ sequencing\ expression =$$

$$(gene\ reading)/(whole\ genome\ reading\ x\ gene\ base\ length)$$

; the gene reading is the RNA sequence reading of the reference gene or the test gene; the whole genome reading is the RNA sequence reading of the whole genome of the reference patient or the test patient; the gene base length is the base length of the reference gene or the test gene.

Preferably, the reference image is one of a CT image, an fMRI image, an X-ray image, an ultrasound image or a pathological tomography image; the test image is one of a CT image, an fMRI image, an X-ray image, an ultrasound image or a pathological tomography image.

Preferably, the cancer is a solid carcinoma.

Another object of the present invention is to provide a system for predicting cancer prognosis, and the system comprises a backbone, which comprises a first computing layer, a second computing layer and a third computing layer, wherein the first computing layer is used to identify a radiomics with a cancer marker. The second computing layer is used to identify a pathological eigenvalue with the cancer marker. The third computing layer integrates the first computing layer and the second computing layer to establish an identification model, wherein the radiomics is the reference radiomics or the test radiomics, and the pathological eigenvalue is the reference pathological eigenvalue or the test pathological eigenvalue. A fourth computing layer is configured for training the backbone to identify the radiomics with changes in cancer prognosis according to the identification model. A fifth computing layer is configured for training the backbone to identify the pathological eigenvalue with changes in cancer prognosis according to the identification model. A fully-connected computing layer with a prognostic index model is configured for integrating the data output by the backbone, the fourth computing layer and the fifth computing layer to calculate a prognostic index, wherein the pathological features comprise genomic features, gene expression, test values or a combination of two or more thereof. The prognostic index model has a mathematical formula as follows:

$$prognostic\ index = \frac{f_2(U_2, X_2)\pi_2}{f_1(U_1, X_1)\pi_1}$$

, wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

Preferably, it further comprises a sixth computing layer used to capture the radiomics and the step of the capturing the radiomics comprises: capturing a lesion image, capturing a plurality of image feature variable values from the lesion image, outputting an image format data, and then normalizing the image format data with a dimensionality reduction matrix to obtain the radiomics. The lesion image is a lesion image of a reference patient or a lesion image of a test patient, and the radiomics is a reference radiomics or a test radiomics, so that the first computing layer can identify the reference radiomics or the fourth computing layer can identify the test radiomics.

In the system as described above, the cancer is a solid carcinoma.

The method for predicting cancer prognosis provided by the present invention has the following advantages:

1. The present invention can achieve cancer prognosis prediction with high accuracy, high sensitivity and high specificity within the scope of a small number of samples. In future applications, it is not only suitable for large-scale cancer prognosis prediction, but also suitable for some diseases with smaller clinical data, to use the method provided by the present invention to carry out prognosis prediction or retrospective research.

2. In the data analysis of colorectal cancer recurrence, the CATCH+ model can obtain higher sensitivity, and can more accurately identify patients with a higher risk of disease recurrence; the accuracy of the CATCH+ model is also higher as measured by F1-Score; however, the specificity of the CATCH model is better than that of the CATCH+ model, whether after rescaling or standardized transformation.

3. In the data analysis of osteoporosis in breast cancer patients, the accuracy of the CATCH+ model is higher than that of the CATCH model for the two type classification problems. In terms of the three type classification problems, the CATCH+ model also has a good discriminative ability, and can identify more osteoporosis patients belonging to a few types.

## BRIEF DESCRIPTION OF THE DRAWINGS

FIG. 1 is a flow chart illustrating the method for predicting cancer prognosis of the present invention.;
FIG. 2A is a flow chart illustrating the process for capturing a reference radiomics;
FIG. 2B is a flow chart illustrating the process for capturing a test radiomics;
FIG. 3A is a block diagram showing a system for predicting cancer prognosis of the present invention;
FIG. 3B is a block diagram showing a system for predicting cancer prognosis in another embodiment of the present invention;
FIG. 4 is a Venn diagram of the intersection of RNA-Seq dataset and image data;
FIG. 5A is a heat map of raw read counts for RNA-seq;
FIG. 5B is a RPKM heat map for RNA-seq;
FIG. 6A is a histogram of raw read counts of the gene NM-000442;
FIG. 6B is a histogram of RPKM of the gene NM-000442;
FIG. 6C is a histogram of the natural logarithm of the RPKM of the gene NM-000442;
FIG. 7A is an original image of a colorectal cross-section of a colorectal cancer patient;
FIG. 7B shows the image format data of the image feature variable data of a colorectal cancer patient after rescaling conversion; and
FIG. 8 shows the image format data of a breast cancer patient's image feature variable data after rescaling conversion.

## DETAILED DESCRIPTION OF THE INVENTION

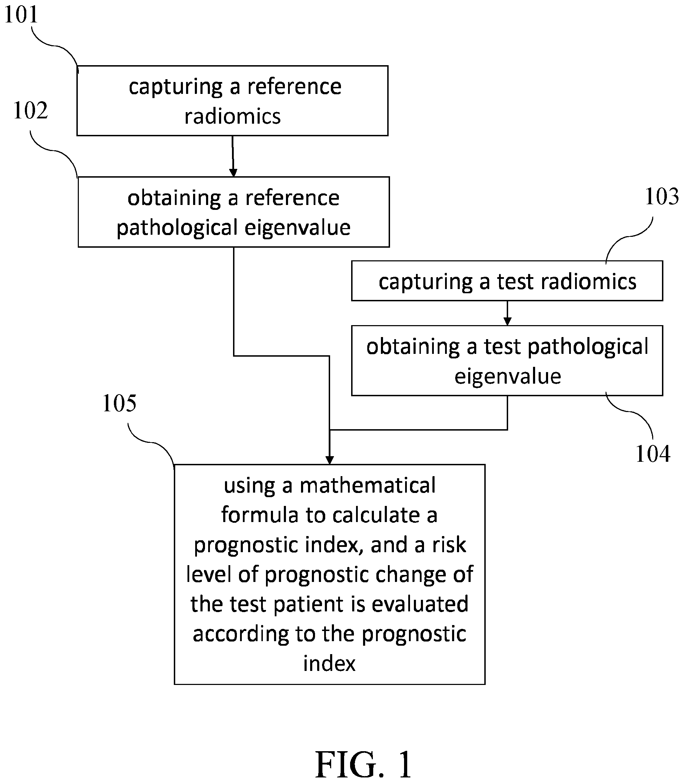

In order to achieve the above-mentioned purpose, one embodiment of the present invention is to provide a method for predicting cancer prognosis. Please refer to FIG. 1, which is a flow chart illustrating a method for predicting cancer prognosis of the present invention. The method comprises:

Step 101: capturing a reference radiomics, wherein the reference radiomics is based on a reference image and the reference image is a lesion medical image of a reference patient;
Step 102: obtaining a reference pathological eigenvalue, wherein the reference pathological eigenvalue is based on pathological features of the reference patient, and the pathological features comprise genomic features, gene expression, test values or a combination of two or more thereof;

Step 103: capturing a test radiomics, wherein the test radiomics is based on a test image and the test image is a lesion medical image of a test patient;
Step 104: obtaining a test pathological eigenvalue, wherein the test pathological eigenvalue is based on pathological features of the test patient, and the pathological features comprise genomic features, gene expression, test values or a combination of two or more thereof; and
Step 105: using a mathematical formula to calculate a prognostic index, wherein a risk level of prognostic change of the test patient is evaluated according to the prognostic index, and the mathematical formula is as follow:

$$prognostic\ index = \frac{f_2(U_2, X_2)\pi_2}{f_1(U_1, X_1)\pi_1}$$

; $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

It should be further noted that this mathematical formula is established based on the CATCH, Covariate-Adjusted Tensor Classification in High Dimensions, model developed by Pan et al. (2019). For details, please refer to "Journal of the American statistical association 114, 527 (2019), 1305-1319"; the difference from the CATCH model established by Pan et al. is that the present invention mainly uses a tensor model, and uses high-dimensional tensor data and covariate to discriminate different types of response variables; in the present invention, there are at least two types, which can handle two or more different variables simultaneously for calculation and classification.
The derivation and establishment of this mathematical formula are carried out below, and the established discriminant is used to judge the level of the prognostic change risk:

1. Assume $Y \in \{1,2\}$ is a type response variable, and $P(Y = k) = \pi_k$, wherein $\sum_{k=1}^{k} \pi_k = 1$, $k = 1, 2$, $X \in R^{p1 \times \dots \times pM}$ is a high-dimensional M-order tensor predictor, and $M \geq 2$, $U \in R^q$ is a q-dimensional covariate, and each subject to the following assumptions:

$$\mathbf{U}|(Y = k) \sim N(\phi_k, \mathbf{\Phi}_k) \quad \dots\dots\dots\dots\dots\dots\dots(1)$$

$$\boldsymbol{X}|(U = u, Y = k) \sim TN(\boldsymbol{\mu}_k + \boldsymbol{\alpha}\bar{\times}_{(M+1)}u, \Sigma_1, \dots, \Sigma_M) \quad \dots\dots\dots\dots(2)$$

, wherein $\phi_k \in R^q$ represents the mean vector of the covariate U under different types k; $\Phi_k \in R^{q \times q}$ represents the covariance matrix of the covariate U under different types k; $\mu_k \in R^{p1 \times \dots \times pM}$ represents the average of the tensor X under different types k after eliminating the effect of the covariate U on X; $\alpha \in R^{p1 \times \dots \times pM \times q}$ represents the relationship between the tensor X and the covariate U; $\Sigma_m \in R^{pm \times pm}$, $m = 1, \dots, M$ represents the common covariance matrix structure of the tensor X under the two types after eliminating the effect of the covariate U on X.

The linear relationship between the tensor X and the covariate U can be expressed by the following formula:

$$\boldsymbol{X}|(Y = k) = \boldsymbol{\mu}_k + \boldsymbol{\alpha}\bar{\times}_{(M+1)}U + \boldsymbol{E} \quad \dots\dots\dots\dots\dots\dots\dots(3)$$

, wherein $E \sim TN(0, \Sigma_1, \dots, \Sigma_M)$. It can be seen from equation (3) that there is a linear relationship between the tensor X and the covariate U, and the coefficient describing the strength of the influence between them is $\alpha$. If the correlation between tensor X and covariate U is ignored, the probability of classification error will increase. Therefore, the influence of the covariate U on X must be eliminated, and the corrected tensor X after eliminating the influence obeys the following distribution:

$$X - \alpha \bar{\times}_{(M+1)} U | (Y = k) \sim TN(\boldsymbol{\mu}_k, \boldsymbol{\Sigma}_1, ..., \boldsymbol{\Sigma}_M) \qquad \ldots\ldots\ldots\ldots (4)$$

2. According to the assumption of the aforementioned 1., the case where the covariate U has different covariance matrix under different types is considered, and the tensor X has the same case for the covariance matrix structure. The goal is to use U and X to discriminate the type of the response variable Y. The best classification effect is achieved by the Bayesian discriminant rule; according to the Bayesian discriminant rule introduced by Härdle and Hlávka (2015), the following discriminant can be established:

$$\hat{Y} = \arg\max_{k=1,2} \pi_k f_k(\boldsymbol{x}, \boldsymbol{u}) \qquad \ldots\ldots\ldots\ldots\ldots\ldots (5)$$

For the aforementioned Bayesian discriminant rule, please refer to Härdle and Hlávka (2015) for details, wherein $f_k(\boldsymbol{x}, \boldsymbol{u})$ represents the joint probability density function of tensor X and covariate U under type k. Under the assumptions of equations (1) and (4), the joint probability density function of (U, X) is:

$$f_k(\boldsymbol{U}, \boldsymbol{X}) = \frac{1}{(2\pi|\boldsymbol{\Phi}_k|)^{1/2}} exp\{-\frac{1}{2}(\boldsymbol{U} - \boldsymbol{\Phi}_k)^T \boldsymbol{\Phi}_k^{-1}(\boldsymbol{U} - \boldsymbol{\Phi}_k)\} \times \frac{1}{(2\pi|\otimes_{m=M}^1 \boldsymbol{\Sigma}_m|)^{1/2}}$$

$$\times exp\{-\frac{1}{2}vec^T(\boldsymbol{X} - \alpha\bar{\times}_{(M+1)}\boldsymbol{U} - \boldsymbol{\mu}_k)(\otimes_{m=M}^1 \boldsymbol{\Sigma}_m)^{-1}$$

$$\times vec(\boldsymbol{X} - \alpha\bar{\times}_{(M+1)}\boldsymbol{U} - \boldsymbol{\mu}_k)\} \qquad \ldots\ldots (6)$$

The discriminant is established by formula (5) and formula (6), and the following is the derivation process. If Y belongs to type 2, it is satisfied as the following formula:

$$\frac{f_2(U_2, X_2)\pi_2}{f_1(U_1, X_1)\pi_1} > 1 \qquad \ldots\ldots\ldots\ldots\ldots\ldots\ldots (7)$$

The natural logarithms of the left side and the right side of the formula (7) are taken at the same time. Since the right side is originally 1, it becomes 0 after taking the natural logarithm. Then, after taking the natural logarithm on the left side, the formula (6) is brought into it, and the derivation process on the left is as follows:

$$ln f_2(\boldsymbol{U}, \boldsymbol{X}) - ln f_1(\boldsymbol{U}, \boldsymbol{X}) + ln(\frac{\pi_2}{\pi_1})$$

$$= [-\frac{1}{2}(\boldsymbol{U} - \boldsymbol{\phi}_2)^T \boldsymbol{\Phi}_2^{-1}(\boldsymbol{U} - \boldsymbol{\phi}_2)] - [-\frac{1}{2}(\boldsymbol{U} - \boldsymbol{\phi}_1)^T \boldsymbol{\Phi}_1^{-1}(\boldsymbol{U} - \boldsymbol{\phi}_1)] +$$

$$[-\frac{1}{2}vec^T(\boldsymbol{X} - \alpha\bar{\times}_{(M+1)}\boldsymbol{U} - \boldsymbol{\mu}_2)(\otimes_{m=M}^1 \boldsymbol{\Sigma}_m)^{-1}] -$$

$$[-\frac{1}{2}vec^T(\boldsymbol{X} - \alpha\bar{\times}_{(M+1)}\boldsymbol{U} - \boldsymbol{\mu}_1)(\otimes_{m=M}^1 \boldsymbol{\Sigma}_m)^{-1}] + ln(\frac{\pi_2}{\pi_1}) + ln(|\boldsymbol{\Phi}_2|^{\frac{1}{2}}) - ln(|\boldsymbol{\Phi}_1|^{\frac{1}{2}})$$

In order to facilitate the arrangement of the formula, let $A = vec(\boldsymbol{X} - \alpha\bar{\times}(M+1)\boldsymbol{U})$ , $B = \otimes_{m=M}^1 \boldsymbol{\Sigma}_m$ , the above formula can be rewritten as:

$$= -\frac{1}{2}\Big\{ [U^T \Phi_2^{-1} U - \phi_2^T \Phi_2^{-1} U - U^T \Phi_2^{-1} \phi_2 + \phi_2^T \Phi_2^{-1} \phi_2] -$$

$$[U^T \Phi_1^{-1} U - \phi_1^T \Phi_1^{-1} U - U^T \Phi_1^{-1} \phi_1 + \phi_1^T \Phi_1^{-1} \phi_1] \Big\} +$$

$$-\frac{1}{2}\Big\{ (A - vec(\mu_2))^T (\otimes_{m=M}^{1} \Sigma_m)^{-1} (A - vec(\mu_2)) -$$

$$(A - vec(\mu_1))^T (\otimes_{m=M}^{1} \Sigma_m)^{-1} (A - vec(\mu_1)) \Big\} + ln(\frac{\pi_2}{\pi_1}) + \frac{1}{2} ln(\frac{|\Phi_1|}{|\Phi_2|})$$

$$= -\frac{1}{2}\Big\{ U^T (\Phi_2^{-1} - \Phi_1^{-1}) U - 2\phi_2^T \Phi_2^{-1} U + 2\phi_1^T \Phi_1^{-1} U + \phi_2^T \Phi_2^{-1} \phi_2 - \phi_1^T \Phi_1^{-1} \phi_1 \Big\} +$$

$$-\frac{1}{2}\Big\{ A^T B^{-1} A - vec^T(\mu_2) B^{-1} A - A^T B^{-1} vec(\mu_2) + vec^T(\mu_2) B^{-1} vec(\mu_2) -$$

$$A^T B^{-1} A - vec^T(\mu_1) B^{-1} A - A^T B^{-1} vec(\mu_1) + vec^T(\mu_1) B^{-1} vec(\mu_1) \Big\} + ln(\frac{\pi_2}{\pi_1}) + \frac{1}{2} ln(\frac{|\Phi_1|}{|\Phi_2|})$$

$$= -\frac{1}{2} U^T (\Phi_2^{-1} - \Phi_1^{-1}) U + (\phi_2^T \Phi_2^{-1} - \phi_1^T \Phi_1^{-1}) U + \frac{1}{2}(\phi_1^T \Phi_1^{-1} \phi_1 - \phi_2^T \Phi_2^{-1} \phi_2) +$$

$$-\frac{1}{2}\Big\{ -2vec^T(\mu_2) B^{-1} A + 2vec^T(\mu_1) B^{-1} A + [vec^T(\mu_2) B^{-1} vec(\mu_2) - vec^T(\mu_1) B^{-1} vec(\mu_1)] \Big\} +$$

$$ln(\frac{\pi_2}{\pi_1}) + \frac{1}{2} ln(\frac{|\Phi_1|}{|\Phi_2|})$$

$$= -\frac{1}{2} U^T (\Phi_2^{-1} - \Phi_1^{-1}) U + (\phi_2^T \Phi_2^{-1} - \phi_1^T \Phi_1^{-1}) U + \frac{1}{2}(\phi_1^T \Phi_1^{-1} \phi_1 - \phi_2^T \Phi_2^{-1} \phi_2) +$$

$$\Big\{ vec^T(\mu_2 - \mu_1) B^{-1} A - \frac{1}{2} vec^T(\mu_2 - \mu_1) B^{-1} vec(\mu_2 + \mu_1) \Big\} + ln(\frac{\pi_2}{\pi_1}) + \frac{1}{2} ln(\frac{|\Phi_1|}{|\Phi_2|})$$

Bringing A and B back to the formula can obtain the following formula:

$$= -\frac{1}{2} U^T (\Phi_2^{-1} - \Phi_1^{-1}) U + (\phi_2^T \Phi_2^{-1} - \phi_1^T \Phi_1^{-1}) U + \frac{1}{2}(\phi_1^T \Phi_1^{-1} \phi_1 - \phi_2^T \Phi_2^{-1} \phi_2) +$$

$$vec^T(\mu_2 - \mu_1)(\otimes_{m=M}^{1} \Sigma_m)^{-1} vec(X - \alpha \bar{\times}_{(M+1)} U) -$$

$$\frac{1}{2} vec^T(\mu_2 - \mu_1)(\otimes_{m=M}^{1} \Sigma_m)^{-1} vec(\mu_2 + \mu_1) + ln(\frac{\pi_2}{\pi_1}) + \frac{1}{2} ln(\frac{|\Phi_1|}{|\Phi_2|})$$

After obtaining the above formula, the result is brought back to the left side of formula (7), when the right side is 0. Finally, the following discriminant is obtained after the above derivation by formula (5) and formula (6):

$$-\frac{1}{2}U^T(\Phi_2^{-1} - \Phi_1^{-1})U + (\phi_2^T\Phi_2^{-1} - \phi_1^T\Phi_1^{-1})U$$

$$+\frac{1}{2}(\phi_1^T\Phi_1^{-1}\phi_1 - \phi_2^T\Phi_2^{-1}\phi_2)$$

$$+ vec^T(\boldsymbol{\mu}_2 - \boldsymbol{\mu}_1)(\otimes_{m=M}^1\Sigma_m)^{-1}vec(\boldsymbol{X} - \boldsymbol{\alpha}\bar{\times}_{(M+1)}U)$$

$$-\frac{1}{2}vec^T(\boldsymbol{\mu}_2 - \boldsymbol{\mu}_1)(\otimes_{m=M}^1\Sigma_m)^{-1}vec(\boldsymbol{\mu}_2 + \boldsymbol{\mu}_1)$$

$$+ ln(\frac{\pi_2}{\pi_1}) + \frac{1}{2}ln(\frac{|\Phi_1|}{|\Phi_2|}) > 0$$

$$\dots\dots(8)$$

When the discriminant (8) has the same covariance matrix under two types of covariate, that is, when $\Phi_1=\Phi_2=\Phi$, the discriminant (8) is simplified as the following formula (9):

$$(\phi_2 - \phi_1)^T\Phi^{-1}U$$

$$-\frac{1}{2}(\phi_2 + \phi_1)^T\Phi^{-1}(\phi_2 - \phi_1)$$

$$+ vec^T(\boldsymbol{\mu}_2 - \boldsymbol{\mu}_1)(\otimes_{m=M}^1\Sigma_m)^{-1}vec(\boldsymbol{X} - \boldsymbol{\alpha}\bar{\times}_{(M+1)}U)$$

$$-\frac{1}{2}vec^T(\boldsymbol{\mu}_2 - \boldsymbol{\mu}_1)(\otimes_{m=M}^1\Sigma_m)^{-1}vec(\boldsymbol{\mu}_2 + \boldsymbol{\mu}_1)$$

$$+ ln(\frac{\pi_2}{\pi_1}) > 0$$

$$\dots\dots(9)$$

The discriminant condition defined according to the Bayesian discriminant rule is as the above formula (8). When the covariance matrix is the same under two types of covariates, the discriminant condition defined in the present invention is as the above formula (9), which is a discriminant model based on the CATCH model; the discriminant formula (8) is based on the extension of the CATCH model, so this discriminant model is called the CATCH+ model. If the data Y meets the conditions of the discriminant, it will be classified as the group to which it belongs. If it is greater than 0, it is judged as the second type, and if it is less than 0, it is judged as the first type; for example, in this embodiment, the first type belongs to the group with a lower risk of prognostic change, and the second type belongs to the group with a higher risk of prognostic change; specifically, prognostic changes comprise relapse, death, sequelae, or the development of other comorbid symptoms.

As can be seen from the above model, the effect is produced from tensor X and covariate U in classification. For the classification effect of U, it consists of two parts. The first part is due to the assumption that U has different covariance matrices under different types, and there is one item, $U^T\left(\Phi_2^{-1} - \Phi_1^{-1}\right)U$, and it is quadratic, which mainly represents the difference caused by the difference between the two types of the covariance matrices. The second part is the coefficient $\left(\phi_2^T\Phi_2^{-1} - \phi_1^T\Phi_1^{-1}\right)$, which mainly represents the difference caused by the difference between the two types after the mean of the covariate U is divided by the covariance matrix. The direct effect of the covariate U on the classification consists of the above two parts. The so-called direct effect means that the classification of Y can be defined by a linear or nonlinear combination of known data observations. In combination with the above-mentioned, it can be found that the direct effect of the covariate U is the same as the quadratic discriminant analysis described by Härdle and Hlávka (2015), and please refer to "Multivariate statistics: exercises and solutions. Springer, 2015" for details.

The coefficient $\alpha$ represents the effect of the covariate U on the tensor X, and also indirectly affects the classification of the response variable Y, which is the indirect effect of the covariate U on the classification. According to the research results of Pan et al. (2019), if the influence of the covariate U on the tensor X is ignored, the probability of misclassification will increase, so it is important to add a coefficient that considers the relationship between X and U in the model. The coefficients $vec^T(\boldsymbol{\mu}_2 - \boldsymbol{\mu}_1)(\otimes_{m=M}^1\Sigma_m)^{-1}$ represent the classification effect of the tensor X on the response variable Y after eliminating the effect of the covariate U.

The mathematical formula can be obtained by the above-mentioned derivation, and the prognostic index can be calculated by the mathematical formula to judge the prognostic change risk level of the test patient. The mathematical formula is as follows:

$$prognostic\ index = \frac{f_2(U_2, X_2)\pi_2}{f_1(U_1, X_1)\pi_1}$$

, wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

For example, the reference radiomics and the test radiomics are extracted by using the pyradiomics suite of python software to capture features from medical image data. The image feature variables are extracted from the lesion medical images provided by the reference patient or the test patient, and some image feature variable values will inevitably be lost, so these image feature variable values will be ignored in the subsequent analysis.

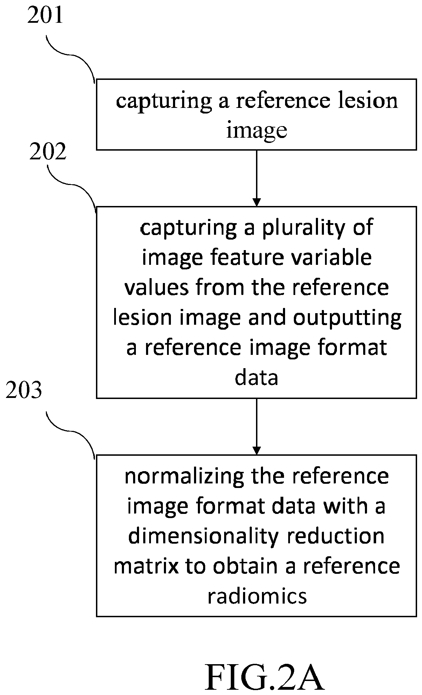

In some embodiments, please refer to FIG. 2A, which illustrates the process of capturing the reference radiomics, and the step 101 comprises:

Step 201: capturing a reference lesion image;
Step 202: capturing a plurality of image feature variable values from the reference lesion image and outputting a reference image format data;
Step 203: normalizing the reference image format data with a dimensionality reduction matrix to obtain the reference radiomics, wherein the reference lesion image is a lesion image of the reference patient.

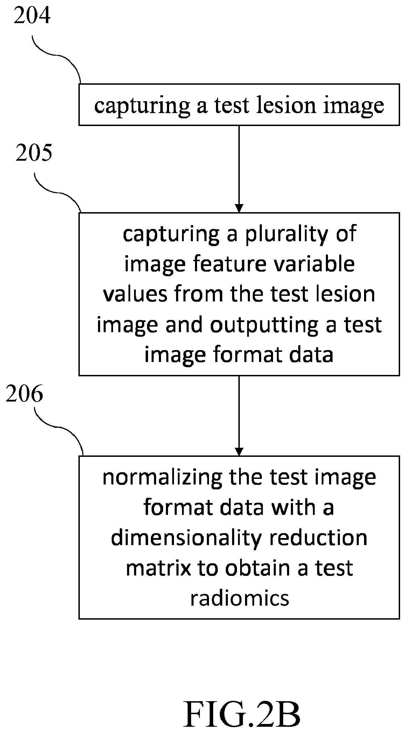

In some embodiments, please refer to FIG. 2B, which illustrates the process of capturing the test radiomics, and the step 103 comprises:

Step 204: capturing a test lesion image;
Step 205: capturing a plurality of image feature variable values from the test lesion image and outputting a test image format data;
Step 206: normalizing the test image format data with a dimensionality reduction matrix to obtain the test radiomics, wherein the test lesion image is a lesion image of the test patient.

It should be noted that the dimensionality reduction matrix is based on the variable screening method carried out by LASSO penalty, and a large number of parameter values are carried out dimensionality reduction; specifically, a plurality of radiomics is converted to output the image format data, the conversion based on the method of Khalifa et al. (2020) converting the obtained reference radiomics and the test radiomics into image format data, and please refer to "IEEE Access 8 (2020), 22874-22883" for details; for example, there are 615 raw read counts of the reference radiomics, and the aforementioned raw read counts are converted and arranged into a 25 by 25 matrix, in which the blanks lacking values are filled with 0; in some preferred embodiments, the reference radiomics and the test radiomics are further normalized, and the normalization adopts two common methods introduced by Chatterjee et al. (2019), which one is standardization, and the other is rescaling; for details, please refer to "IEEE Transactions on Radiation and Plasma Medical Sciences 3, 2 (2019), 210-215"; after standardization of each radiomics, its distribution is transformed into a mean of 0 and a standard deviation of 1, and rescaling transforms the range of each radiomics into a range between 0 and 1.

In some embodiments, the gene expression comprises RNA sequencing amount or protein expression; for example, the amount of RNA sequencing is determined by polymerase chain reaction (PCR), qPCR, qRT-PCR, RNA-seq, microarray analysis, SAGE, MassARRAY technology, next-generation sequencing, or FISH; protein expression is quantitatively analyzed for specific proteins by methods such as Western blot, immunohistochemistry (IHC) or immunoprecipitation (IP).

In some preferred embodiments, the genomic features comprise gene copy number, gene mutant site or single nucleotide polymorphisms (SNPs).

In some embodiments, when the gene expression is an RNA sequencing, the method further comprises: normalizing a gene reading with a following formula to obtain the RNA sequencing expression:

$$\text{RNA sequencing expression} = \frac{\text{gene reading}}{\text{whole genome reading x gene base length}}$$

, wherein the gene reading is the RNA sequence reading of the reference gene or the test gene; the whole genome reading is the RNA sequence reading of the whole genome of the reference patient or the test patient; the gene base length is the base length of the reference gene or the test gene.

It should be noted that in the RNA sequencing results, and it can be found that many genes belong to low expression. When gene expression is measured, the number of gene sequences will be affected by gene length and sequencing depth. Because the sequencing process is a random sampling process, genes with longer sequences are more likely to be selected than genes with shorter sequences. If the gene sequence length and sequencing depth are ignored, the true expression of the gene will be misestimated so as to result in a decrease in the accuracy of the discriminant results. Therefore, in this embodiment, the number of gene sequences is normalized and converted into RPKM (Reads Per Kilobase Million).

In some embodiments, the test value is a biochemical value or a physical examination value; specifically, the biochemical value can be derived from routine biochemical tests such as blood, urine, feces, etc., such as red blood cells, white blood cells, platelets, heme, blood cells Volume, leukocyte classification, urine pH, specific gravity, urine sugar, urine protein, leukocytes and urine occult blood, and fecal occult blood or parasite examination, etc.

In other embodiments, biochemical tests for specific organs such as liver, kidney, cardiovascular, etc. are also specific data sources for biochemical values in this embodiment, such as liver function, kidney function, metabolism, for example, liver functional tests such as B liver surface antigen and antibody, C liver surface antibody albumin, total protein, total bilirubin, alkaline phosphatase, glutamine oxalate transaminase (AST/GOT), alanine amino acid transaminase (ALT) /GPT, γ-glutamate transferase (y-GT), direct bilirubin, etc.; kidney function tests are such as blood urea nitrogen, creatinine, total cholesterol, triglycerides, high/low density lipoprotein, heart disease risk factors, thyrotropin, uric acid, blood glucose before meals, glycosylated hemoglobin, free tetraiodothyronine, etc.; cardiovascular tests are such as high-sensitivity C-reactive protein, homocysteine, and type A lipoprotein determination.

In some preferred embodiments, the test value is a physical examination value; the physical examination is a method for medical personnel to use their own senses, inspection equipment or laboratory instruments to directly or indirectly understand the patient's physical condition to collect the patient's current objective pathological data covering vital signs, state of consciousness, head and neck, eyes ENT, chest, abdomen, urology, reproduction, skeletal muscles, peripheral blood vessels, neurology, etc.; for example, eye, ear, nose and throat examinations are such as vision, color discrimination, hearing, etc.; chest examination is such as breast, lung, cardiovascular examination, etc.; abdominal examination is such as liver, spleen, kidney, gastrointestinal examination, etc.; musculoskeletal examination is such as musculoskeletal appearance, bone density measurement, musculoskeletal examination Functions such as muscle strength, joint function, etc.; neurological examinations is such as cognitive function, state of consciousness, cranial nerves, spinal nerves and reflex examinations, etc.

In some embodiments, the reference image is one of a CT image, an fMRI image, an X-ray image, an ultrasound image or a pathological tomography image; the test image is one of a CT image, an fMRI image, an X-ray image, an ultrasound image or a pathological tomography image.

In some embodiments, the cancer is a solid carcinoma, such as colorectal cancer, liver cancer, breast cancer, stomach cancer, esophageal cancer, oral cancer, or brain cancer.

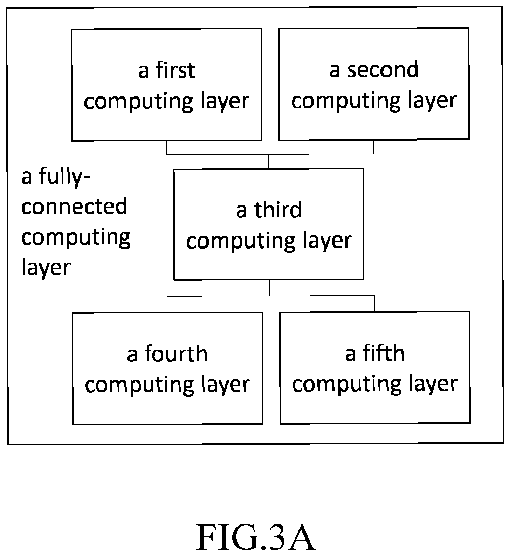

Another embodiment of the present invention is a system for predicting cancer prognosis, please refer to FIG. 3A, which presents a block diagram of a cancer prognosis prediction system of the present invention, and the prediction system comprises a backbone, which comprising a first computing layer, a second computing layer and a third computing layer, wherein the first computing layer is used to identify a radiomics with a cancer marker. The second computing layer is used to identify a pathological eigenvalue with the cancer marker. The third computing layer integrates the first computing layer and the second computing layer to establish an identification model, wherein the radiomics is the reference radiomics or the test radiomics, and the pathological eigenvalue is the reference pathological eigenvalue or the test pathological eigenvalue. A fourth computing layer is configured for training the backbone to identify the radiomics with changes in cancer prognosis according to the identification model. A fifth computing layer is configured for training the backbone to identify the pathological eigenvalue with changes in cancer prognosis according to the identification model. A fully-connected computing layer with a prognostic index model is configured for integrating the data output by the backbone, the fourth computing layer and the fifth computing layer to calculate a prognostic index, wherein the pathological features comprise genomic features, gene expression, test values or a combination of two or more thereof. The prognostic index model has a mathematical formula as follows:

$$prognostic\ index = \frac{f_2(U,X)\pi_2}{f_1(U,X)\pi_1}$$

, wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

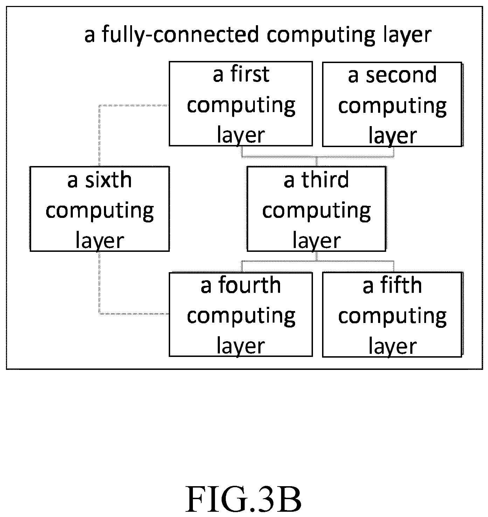

In some embodiments, please refer to FIG. 3B, the prediction system further comprises a sixth computing layer used to capture the radiomics, and the step of the capturing the radiomics comprises: capturing a lesion image, capturing a plurality of image feature variable values from the lesion image, outputting an image format data, and then normalizing the image format data with a dimensionality reduction matrix to obtain the radiomics. The lesion image is a lesion image of a reference patient or a lesion image of a test patient, and the radiomics is a reference radiomics or a test radiomics, so that the first computing layer can identify the reference radiomics or the fourth computing layer can identify the test radiomics.

The prediction system as above, wherein the cancer is a solid carcinoma, such as colorectal cancer, liver cancer, breast cancer, stomach cancer, esophageal cancer, oral cancer, or brain cancer.

Embodiment 1

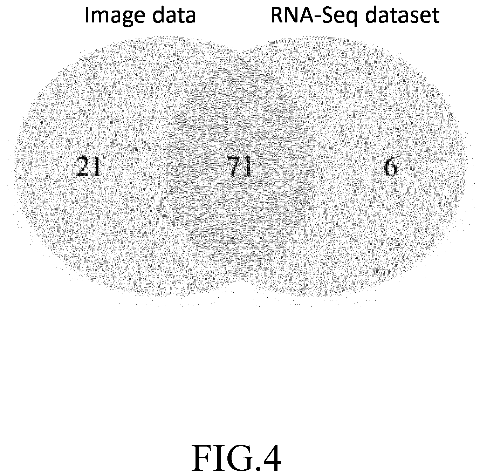

In this embodiment, there are a total of three different data sets, the first of which is clinical data to record the personal information of 77 patients; the second is RNA sequencing data to record the read count of gene sequences of 77 patients, and the clinical data and RNA data of 77 patients are the same; the third is the imaging data of 92 patients who received computed tomography scans. Figure 4 shows the Venn diagram formed by the intersection of the RNA-Seq data sets and the image data. It can be seen from the above Venn diagram that there are 71 patients with both RNA data and imaging data, and in the subsequent analysis of this embodiment, these 71 patients will be the main ones.

Table 1

| type | variable | illustrate | coding |
|---|---|---|---|
| type | Sample ID | Medical record number | - |
| | RFS status | Whether the disease recurs | Yes=yes , NO=no |
| | OS status | Survive or not | Yes=yes , NO=no |
| | Gender | sex | Female=female Male=male |
| continuous | RFS | Disease recurrence time or total follow-up time | Unit: day |
| | OS | overall survival time | Unit: day |
| | Age.when.diagnosis | age at diagnosis | Unit: year |

The personal information of 71 patients with RNA-Seq data is recorded in the clinical data. The variable introduction is shown in Table 1, which lists the type, interpretation and description, and the encoding method of each variable separately.

Table 2

| variable | level | sample number (%)/mean (standard deviation) |
|---|---|---|
| Whether the disease recurs | yes | 21 (29.57) |
| | no | 50 (70.43) |
| Survive or not | survive | 67 (94.36) |
| | die | 4 (5.64) |
| Sex | female | 36 (50.70) |
| | male | 35 (49.30) |

(continued)

| variable | level | sample number (%) /mean (standard deviation) |
|---|---|---|
| Disease recurrence time or total follow-up time | - | 1243.65 (84) |
| overall survival time | - | 1416.78 (737.38) |
| age at diagnosis | - | 56.76 (11.36) |

Table 2 lists the basic descriptive statistics of each variable. If the variable is a discrete variable, the number of samples and percentages of this variable at each level are listed. If the variable is a continuous variable, the mean and standard deviation of the variable are listed. It can be seen from Table 2 that 21 of the 71 patients have colorectal cancer recurrence, and the disease recurrence rate is about 29.57%.

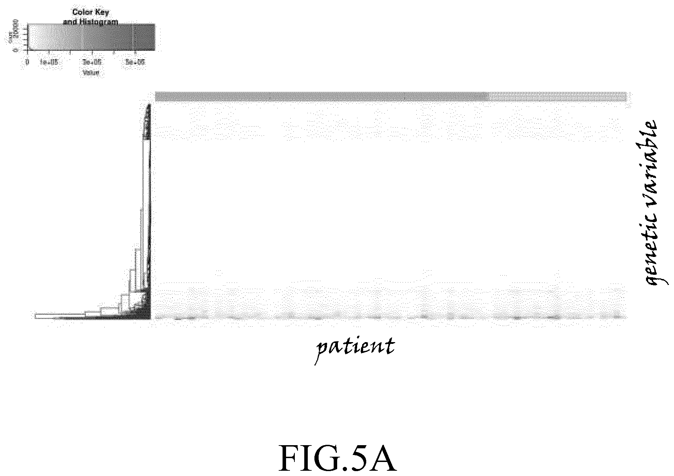

The RNA-Seq data originally records the read counts of gene sequences of 398 genes in 77 patients to represent the expression of the gene, which also known as the raw read counts, and there are 71 people have image data at the same time. Please refer to Figure 5A, which is a heat map of raw read counts by RNA-seq, in which the horizontal axis represents patients and the vertical axis represents genetic variables; the part of the horizontal axis is divided into green group to represent colorectal cancer recurrence and the orange group to represent no colorectal cancer recurrence. As shown in Figure 5A, since most genes are low expression level, in order to prevent the number of gene sequences from being affected by gene length and sequencing depth, the number of gene sequences is converted into RPKM (Reads Per Kilobase Million) by the following formula:

$$RNA\ sequencing\ expression = \frac{genetic\ reads}{Whole\ genome\ reads\ X\ gene\ base\ length}$$

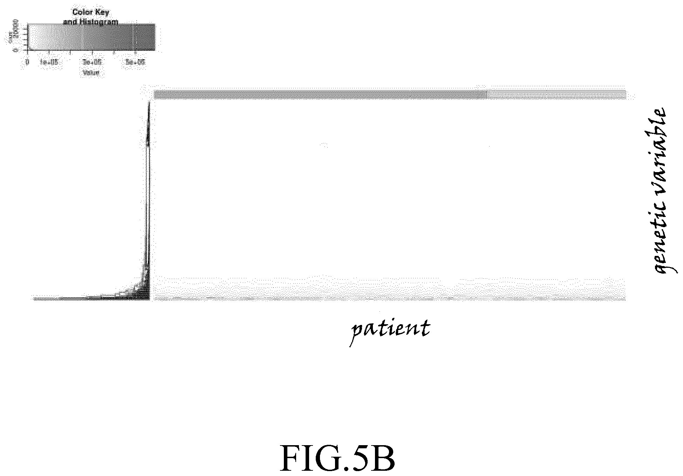

Please refer to Figure 5B, which is an RPKM heat map of RNA-seq. It can be found that the reads and expressions of most genes are extremely rare. In order to avoid too many gene variables unrelated to the recurrence of colorectal cancer entering the model and affecting the subsequent analysis, the differential expression analysis of genes is performed first, and the top 10 genes with significant differential expression are selected. First, genes with sum of reads in all patients below 1000 are deleted, and then differential expression analysis is performed using the DESeq2 R package. The assumption for testing each gene is as follow:

$H_0$: There is no differential expression across the two sample groups. (LFC = 0)
$H_1$: There is differential expression across the two sample groups. (LFC = 0)

In the above test, the two groups refer to one group with recurrence of colorectal cancer and the other group without recurrence of colorectal cancer. The Log2 fold changes (LFC) represents how much the corresponding gene expression changes in patients with recurrence compared with patients without recurrence. If it is equal to 0, there is no difference in the expression of the gene between the two groups. Finally, by sorting the p-values corrected by the BH method from large to small, the top 10 representative genes are selected for subsequent analysis, and in this embodiment, the genes comprise CCR5, IFI35, CXCR4, TLR7, PECAM1, PRDM1 and other colorectal cancer related genes.

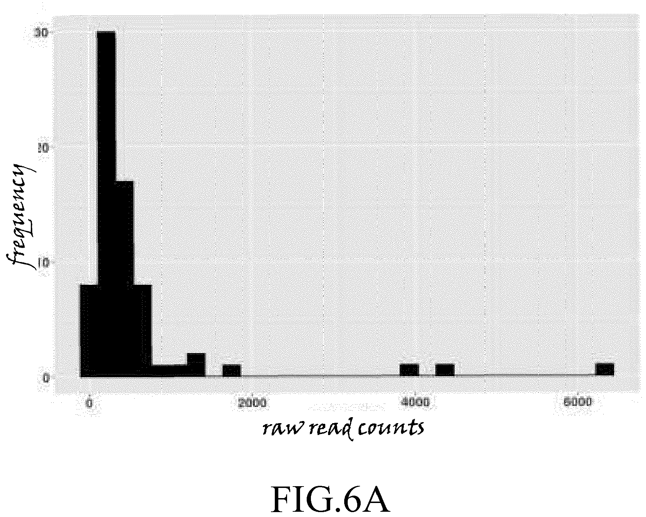

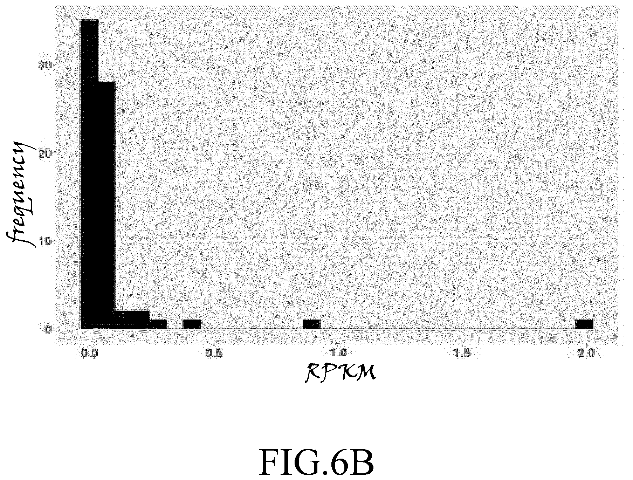

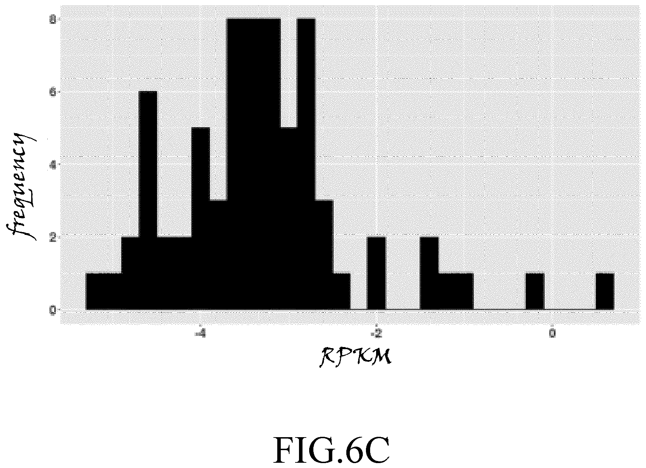

Please refer to FIG. 6A to FIG. 6C, which are histograms illustrating the distribution of gene expression; in order to make the distribution of gene expression clearer, in the present embodiment, the gene with the smallest p-value after correction is used as the observation object in the gene expression difference analysis; as shown in Figure 6A, the histogram of the raw read counts of the gene NM-000442, which is a human platelet and endothelial cell adhesion molecule 1 (PECAM1), shows the only extreme left skewness histogram of the raw read counts in the results of this RAN-seq; as shown in Figure 6B, the histogram of the RPKM of the gene NM-000442, it can be seen that the normalized RPKM of NM-000442 will form an extremely right skewness data; therefore, the natural logarithm of the RPKM of the aforementioned gene NM-000442 is taken to form a relatively symmetrical distribution, as shown in Figure 6C.

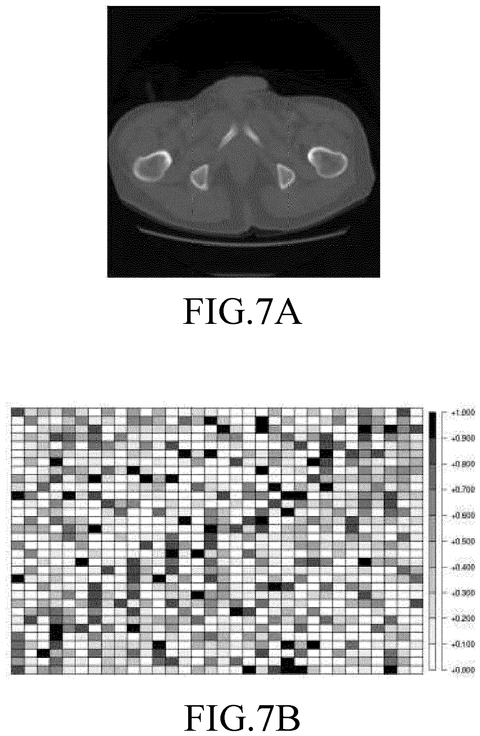

The imaging data originally records the imaging data of 92 people after CT scan, and 71 of them also have RNA-seq data at the same time. Please refer to Figure 7A, which is the original image of the colorectal cross-section of one of the patients; the feature extraction is performed on the original image with pyradiomics of python software to obtain 1037 feature variables , and the aforementioned feature variables are arranged into 33 by 32 matrix, wherein the blanks lacking variable values in the matrix are filled with 0; next, normalization and rescaling are performed for the feature variables; please refer to FIG. 7B , which is the result of converting the radiomics of the aforementioned patient into image format data and applying rescaling and conversion, and the value range of each feature variable is converted into a range between 0 and 1.

Similar to the aforementioned radiomics processing method, in this embodiment, feature variable values capture is performed for the computed tomography images of the other 71 patients.

The radiomics obtained from the CT images of the aforementioned 71 patients is converted into image format data, and this image format data is used as tensor data X. The screened 10 gene variables are converted to RPKM or log (RPKM) as covariate data U to discriminate the changes in patient prognosis; in this embodiment, the change in prognosis is to determine whether the colorectal cancer recurs.

The aforementioned image format data is divided into a training set of 70% and a test set of 30%, and the training set will maintain the original disease recurrence rate of 0.29. By repeating 100 times, the sorted average measurement indexes comprise "sensitivity", "specificity", " accuracy" and "recall", and the discriminant results are shown in Table 5.

It should be further explained that "sensitivity" refers to the ratio of being correctly judged to be positive among all actually positive individuals; "specificity" means the ratio of being correctly judged to be negative among all actually negative individuals; therefore, a test with high sensitivity and high specificity means that the test can correctly determine the target of interest and rarely misjudge other types as the target of interest. When a classification model of unbalanced data is usually done, the model easily tends to judge an individual as positive or negative. For any test, there is a trade-off between sensitivity and specificity. At this time, in order to more accurately measure the effect of the model, another set of index "accuracy" and "recall" are usually considered. "Accuracy" means the ratio of being actually positive among all individuals who are correctly judged to be positive. "Recall" is synonymous with sensitivity. It is usually hoped that the accuracy and recall of a model are not too bad, so the F1-Score index is used as a measure index of imbalanced classification problems. The F1-Score index pays attention to both accuracy and recall, which can reflect the accuracy of the model in a balanced manner.

In the aspect of parallel comparison, in the present embodiment, a linear discriminant analysis (LDA) model is simultaneously used to discriminate the recurrence of colorectal cancer with tensor or gene data to compare the prediction models provided by the present invention. Please refer to Table 3, which is judged based on tensor data, that is, judged by image format data; Table 4 is based on the genetic variable RPKM for discrimination, that is, based on the 10 genetic variables for discrimination after screening.

Table 3

| tensor | rescaling | standardized |
|---|---|---|
| accuracy | 0.567 | 0.596 |
| sensitivity | 0.285 | 0.274 |
| specificity | 0.696 | 0.735 |
| F 1-score | 0.293 | 0.303 |

Table 4

| genetic variable | RPKM | Log(RPKM) |
|---|---|---|
| accuracy | 0.6238 | 0.6302 |
| sensitivity | 0.3785 | 0.2720 |
| specificity | 0.7389 | 0.7939 |
| F1-score | 0.3659 | 0.3043 |

Please refer to Table 5, it can be seen that the accuracy of the CATCH+ model is close to the accuracy of the CATCH model. But if the standardized transformation is used for tensor data and genetic variables are used in the form of RPKM, the CATCH+ model can get a sensitivity of 0.5778. That is, the proportion of recurrence can be accurately judged, which is higher than the CATCH model of 0.3963; and the specificity of the CATCH model is better than that of the CATCH+ model.

Table 5

| | tensor | rescaling | | standardized | |
|---|---|---|---|---|---|
| | covariate | RPKM | Log (RPKM) | RPKM | Log (RPKM) |
| accuracy | CATCH | 0.6559 | 0.6381 | 0.6633 | 0.6509 |
| | CATCH+ | 0.6726 | 0.6727 | 0.6746 | 0.6904 |
| sensitivity | CATCH | 0.3813 | 0.3869 | 0.3963 | 0.4217 |
| | CATCH+ | 0.3820 | 0.1964 | 0.5778 | 0.2722 |
| specificity | CATCH | 0.7767 | 0.7543 | 0.7788 | 0.7522 |
| | CATCH+ | 0.7788 | 0.8802 | 0.7247 | 0.8050 |
| F1-score | CATCH | 0.7602 | 0.7412 | 0.7634 | 0.7501 |
| | CATCH+ | 0.7698 | 0.7915 | 0.7706 | 0.7972 |

Through Tables 3 to 5, the tensor cooperating with covariate CATCH and CATCH+ models used in the results of colorectal cancer recurrence discrimination, in addition to sensitivity, whether in terms of accuracy, specificity or F1-score, has much better performance than LDA model; in taking the tensor processed by rescaling as an example, the accuracy is CATCH (0.6559, 0.6381), CATCH+ (0.6726, 0.6727) respectively, and the LDA model is only 0.567; the specificity is CATCH (0.7767, 0.7543), CATCH+ (0.7788, 0.8802) respectively, and the LDA model is only 0.696; the F1-score is significantly improved compared to the LDA model of 0.293 and is CATCH (0.7602, 0.7412), CATCH+ (0.7698, 0.7915) respectively; in taking gene variables in RPKM as an example, the accuracy is CATCH (0.6559, 0.6633), CATCH+ (0.6726, 0.6746) respectively, and the LDA model is 0.6238; the F1-score is CATCH (0.7602, 0.7634), CATCH+ (0.7698, 0.7706) respectively, and the LDA model is only 0.3659. It can be seen from the above analysis results that the prediction model provided by the present invention can obtain better discrimination results as a whole by combining image format data and gene variables for covariate analysis in the discrimination results of colorectal cancer recurrence.

Embodiment 2

In the present embodiment, the purpose to detect whether the breast cancer patient suffers from osteoporosis is to determine whether there is osteoporosis; there are mainly two data sets, the first one is clinical data, which records the patient's basic personal data and the patient's bone determination results. The second one is the Radiomics data after the computer tomography scan and feature extraction, that is, the image feature variable data. The data is divided into training data set and test data set, with 313 and 99 people respectively. Table 6 describes the clinical data and imaging feature variable data, and lists the type, interpretation and description, and coding method of each variable.

In the training data set of clinical data, the personal information and bone density test data of 313 patients with breast cancer are recorded. The variables are introduced in Table 6. Please refer to Table 7, which lists the basic descriptive statistics of each variable. It is listed according to the following principles: if it is a type variable, the number of samples and percentages of this variable at each level are listed; if the variable is a continuous variable, then the mean and standard deviation of the variable are listed.

Table 6

| pattern | variable | illustrate | coding |
|---|---|---|---|
| type | Chart. no | patient number | - |
| | Gender | patient sex | 0=female |
| | DEXA.L1.bone.health | Bone determination results | 0: health<br>1: osteopenia<br>2: osteoporosis |

(continued)

| pattern | variable | illustrate | coding |
|---|---|---|---|
| continuous | Age | age at bone density test | |
| | BH cm | patient height | |
| | BW kg | patient weight | |
| | DEXA.L1.T.score | T-score after bone density test results compared to young adults | |

Table 7

| | variable | level | sample number (%) /mean (standard deviation) |
|---|---|---|---|
| Training data Set | patient sex | female | 313(100%) |
| | Bone determination results | Normal Osteopenia Osteoporosis | 145(46.33) 133(42.49) 35(11.18) |
| | age at bone density test | | 60.66(9.57) |
| | patient height | | 155.59(5.92) |
| | patient weight | | 58.26(9.59) |
| | T-score after bone density test results compared to young adults | | -1.02(1.3) |
| Test Data Set | patient sex | female | 99(100%) |
| | Bone determination results | Normal Osteopenia Osteoporosis | 41(41.42) 48(48.48) 10(10.10) |
| | age at bone density test | | 64.22(11.12) |
| | patient height | | 155.71(5.83) |
| | patient weight | | 60.04(11.23) |
| | T-score after bone density test results compared to young adults | | -1.13(1.22) |

The determination of osteoporosis is mainly based on the T-score value calculated by comparing the detected bone density with the young and same-sex; when the T-score value is greater than or equal to -1.0, it is judged as normal bone mass (normal); when the T-score value is between -1.0 and -2.5, it is judged as osteopenia; when it is equal to or less than -2.5, it is judged as osteoporosis; please refer to Table 7, among the 313 breast cancer patients in the training data set, about 42.49% of them are osteopenia and 11.18% are osteoporosis.

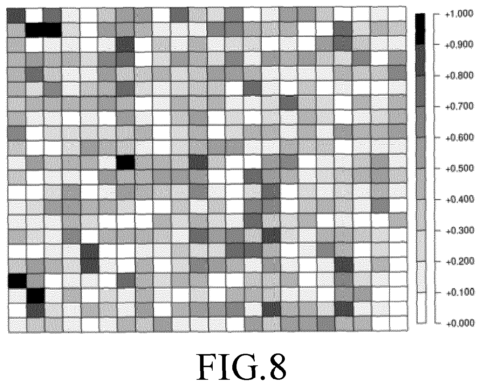

The image feature variable data records the data of 313 people after CT scan and extraction of feature variables through pyradiomics of python software. There are 479 image feature variables in total; among them, there are 2 feature variables whose values are all 0, so there are 477 feature variables in total after ignoring these two variables. For the above 477 feature variables, they are arranged into a 22-by-22 matrix, and the blanks for the lack of variable values in the matrix are filled with 0. Please refer to Figure 8, which is the result of rescaling the image format data converted from the image feature variable data of one breast cancer patient.

For clinical data, age and weight are selected as covariate data U; in this embodiment, there are two classification methods, one is to treat patients with osteopenia and osteoporosis as one type, and compare them with patients with normal bone density. The other is to perform three type discriminations to discriminate between normal bone mass, osteopenia and osteoporosis by using image format data and clinical data.

The data of 313 people is used as a training data set to build a model, and the test data set of 99 people is inputted into the model to evaluate the discriminative effect of the model. It can be seen from Table 8 that, for any conversion method, including rescaling or standardized, the accuracy shown by the CATCH+ model is higher than that of the CATCH model, wherein the highest accuracy (0.737) can be obtained after the image feature variables are standardized; the highest sensitivity (0.862) can be obtained if the image feature variables are not transformed in any way, patients with osteopenia or osteoporosis having the highest rate of being accurately judged are shown under the prediction of the CATCH+ model; the highest F1-Score values are also obtained without any transformation of the image feature variables. The CATCH+ model is superior to the CATCH model in terms of accuracy, sensitivity, specificity and F1-Score in the discrimination of osteopenia or osteoporosis in breast cancer patients; in addition, the highest accuracy, sensitivity and F1-Score can be obtained by using the CATCH+ model to discriminate under the feature variables without any transformation.

Table 8

| tensor | model type | unconverted | rescaling | standardized |
|---|---|---|---|---|
| accuracy | CATCH | 0.717 | 0.676 | 0.686 |
| | CATCH+ | 0.727 | 0.707 | 0.737 |
| sensitivity | CATCH | 0.844 | 0.741 | 0.706 |
| | CATCH+ | 0.862 | 0.741 | 0.758 |
| specificity | CATCH | 0.536 | 0.585 | 0.658 |
| | CATCH+ | 0.536 | 0.658 | 0.707 |
| F1-score | CATCH | 0.777 | 0.728 | 0.725 |
| | CATCH+ | 0.787 | 0.747 | 0.771 |

Next, there are the results of the three type discriminations in this embodiment; please refer to Tables 10 to 12, which are obtained after performing different standardized transformations on the image feature variables, and the result of the confusion matrix can be obtained by using the CATCH and CATCH+ models; confusion Matrix, after each data is classified by the model, whose classification result will be classified into one of the four states shown in Table 9. The definition of true positive is that the model judgment result is true and the real condition is true, and the definition of other states can be deduced by analogy.

Table 9

| | | Model classification | |
|---|---|---|---|
| | | judged to be true | judged to be false |
| Real condition | true | true positive | false negative |
| | false | false positive | true negative |

Please refer to Table 10, which is the three type discrimination results of breast cancer osteoporosis whose image feature variables are not transformed (Original). In Table 10, there are 25 patients who are judged by the CATCH+ model to be normal bone quality and the results judged by the model are also normal bone quality, and these 25 people account for 25.25% of the total 99 patients. The percentages of the main diagonal line are summed up to the accuracy. On the premise that the image feature variables are not transformed, the judgment accuracy of the CATCH model is 0.6061, and that of the CATCH+ model is 0.6263.

Table 10

| | CATCH | CATCH+ | | | |
|---|---|---|---|---|---|
| accuracy | 0.6061 | 0.6263 | | | |
| | actual type | | | | |
| model identificatio n results | normal bone | osteopeni a | osteoporosi s | normal bone | osteopeni a | osteoporosi s |
| normal bone | 25 (25.25% ) | 15 (15.15%) | 1 (1.01%) | 27 (27.27% ) | 10 (10.10%) | 1 (1.01%) |

(continued)

| model identification results | actual type | | | | | |
|---|---|---|---|---|---|---|
| | normal bone | osteopeni a | osteoporosi s | normal bone | osteopeni a | osteoporosi s |
| osteopenia | 16 (16.16% ) | 33 (33.33%) | 7 (7.07%) | 14 (14.14% ) | 34 (34.34%) | 8 (8.08%) |
| osteoporosis | 0 (0.00%) | 0 (0.00%) | 2 (2.03%) | 0 (0.00%) | 4 (4.04%) | 1 (1.02%) |

Please refer to Table 11, which is the three type discrimination results of breast cancer osteoporosis whose image feature variables are standardization. On the premise that the image feature variables are standardization, the judgment accuracy of the CATCH model is 0.6162, and the judgment accuracy of the CATCH+ model is 0.6465.

Table 11

| | CATCH | CATCH+ | | | | |
|---|---|---|---|---|---|---|
| accuracy | 0.6162 | 0.6465 | | | | |
| | actual type | | | | | |
| model identification results | normal bone | osteopeni a | osteoporosi s | normal bone | osteopeni a | osteoporosi s |
| normal bone | 28 (28.28% ) | 15 (15.15%) | 3 (3.03%) | 29 (29.29% ) | 14 (14.14%) | 3 (3.03%) |
| osteopenia | 13 (13.13% ) | 32 (32.32%) | 6 (6.06%) | 12 (12.12% ) | 32 (32.32%) | 4 (4.04%) |
| osteoporosis | 0 (0.00%) | 1 (1.01%) | 1 (1.02%) | 0 (0.00%) | 2 (2.02%) | 3 (3.04%) |

Please refer to Table 12, which is the three type discrimination results of breast cancer osteoporosis by rescaling the image feature variables. On the premise that the image feature variables are rescaled, the determination accuracy of the CATCH model is 0.6263, and the judgment accuracy of the CATCH+ model is 0.6263.

Table 12

| | CATCH | CATCH+ | | | | |
|---|---|---|---|---|---|---|
| accuracy | 0.6263 | 0.6263 | | | | |
| | actual type | | | | | |
| model identification results | normal bone | osteopeni a | osteoporosi s | normal bone | osteopeni a | osteoporosi s |
| normal bone | 25 (25.25% ) | 13 (13.13%) | 2 (2.02%) | 28 (28.28% ) | 13 (13.13%) | 3 (3.03%) |
| osteopenia | 16 (16.16% ) | 35 (35.35%) | 6 (6.06%) | 13 (13.13% ) | 31 (31.31%) | 4 (4.04%) |
| osteoporosis | 0 (0.00%) | 0 (0.00%) | 2 (2.03%) | 0 (0.00%) | 4 (4.04%) | 3 (3.03%) |

From the foregoing results, it can be found that the CATCH+ model obtains the same accuracy as the CATCH model after the image feature variables are transformed by rescaling. It can get higher accuracy without any transformation (original) or standardization transformation. Among them, for a few types of osteoporosis, the CATCH+ model can discriminate 3 people by using standardization transformation, which proves that the CATCH+ model is better than the CATCH model in the three type classification problems.

The method for predicting cancer prognosis provided by the present invention can simultaneously use high-dimensional tensor data and covariate to perform discriminant analysis, and further comprise the condition that the covariate in the covariance matrix is different under different types. At the same time, after correcting the linear relationship between tensor and covariate, a CATCH+ model is established for type discrimination, which can be applied to classification discrimination of three or more types.

In the cancer prognosis prediction method provided by the present invention, in the data analysis of colorectal cancer recurrence, the CATCH+ model can obtain higher sensitivity, and can more accurately identify patients with a higher risk of disease recurrence. And the accuracy of CATCH+ is also higher through the measurement of F1-Score. However, the CATCH model outperforms the CATCH+ model in specificity, whether after rescaling or standardized transformation.

In the cancer prognosis prediction method provided by the present invention, in the data analysis of osteoporosis in breast cancer patients, the accuracy of the CATCH+ model is higher than that of the CATCH model in terms of two type classification problems. In terms of the three type classification problems, the CATCH+ model also has a good discriminative ability, and can identify more osteoporosis patients belonging to a few types.

The cancer prognosis prediction method provided by the present invention can achieve the cancer prognosis prediction with high accuracy, high sensitivity and high specificity within the scope of a small number of samples, In the future applications, it is not only suitable for large-scale cancer prognosis prediction, but also suitable for some diseases with smaller scale of clinical data. The method provided by the present invention is used for prognosis prediction or retrospective research.

It is to be understood that the foregoing descriptions of the embodiments are given by way of example only, and various modifications may be made by those skilled in the art to which this field pertains. The above specification and examples provide a complete description of the flow of exemplary embodiments of the invention and their uses. Although the above embodiments disclose specific embodiments of the present invention, they are not intended to limit the present invention.

**Claims**

1. A method for predicting cancer prognosis, the method comprising:

   capturing a reference radiomics, wherein the reference radiomics is based on a reference image and the reference image is a lesion medical image of a reference patient;
   obtaining a reference pathological eigenvalue, wherein the reference pathological eigenvalue is based on pathological features of the reference patient, the pathological features comprising genomic features, gene expression, test values or a combination of two or more thereof;
   capturing a test radiomics, wherein the test radiomics is based on a test image and the test image is a lesion medical image of a test patient;
   obtaining a test pathological eigenvalue, wherein the test pathological eigenvalue is based on pathological features of a test patient, the pathological features comprising genomic features, gene expression, test values or a combination of two or more thereof; and
   using a mathematical formula to calculate a prognostic index, and a risk level of prognostic change of the test patient is evaluated according to the prognostic index, the mathematical formula as follow:

   $$prognostic\ index = \frac{f_2(U_2, X_2)\pi_2}{f_1(U_1, X_1)\pi_1}$$

   , wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

2. The method according to claim 1, wherein the step of the capturing the reference radiomics comprising: capturing a reference lesion image, capturing a plurality of image feature variable values from the reference lesion image, outputting a reference image format data, and then normalizing the reference image format data with a dimensionality reduction matrix to obtain a reference radiomics, wherein the reference lesion image is a lesion image of the reference patient.

3. The method according to any of claims 1 or 2, wherein the step of the capturing the test radiomics comprises: capturing a test lesion image, capturing a plurality of image feature variable values from the test lesion image, outputting a test image format data, and then normalizing the test image format data with a dimensionality reduction matrix to obtain a test radiomics, wherein the test lesion image is a lesion image of the test patient.

4. The method according to any of claims 1 to 3, wherein the gene expression comprises RNA sequencing expression or protein expression.

5. The method according to any of claims 1 to 4, wherein the genomic features comprise gene copy number, gene mutant site, and single nucleotide polymorphisms ,SNPs.

6. The method according to any of claims 4 or 5, wherein when the gene expression is an RNA sequencing, the method further comprises:
normalizing a gene reading with a following formula to obtain the RNA sequencing expression:

$$RNA\ sequencing\ expression = \frac{gene\ reading}{whole\ genome\ reading\ x\ gene\ base\ length}$$

, wherein the gene reading is the RNA sequence reading of the reference gene or the test gene; the whole genome reading is the RNA sequence reading of the whole genome of the reference patient or the test patient; the gene base length is the base length of the reference gene or the test gene.

7. The method according to any of claims 1 to 6, wherein the reference image is one of a CT image, an fMRI image, an X-ray image, an ultrasound image or a pathological tomography image; the test image is one of a CT image, an fMRI image, an X-ray image, an ultrasound image or a pathological tomography image.

8. The method according to any of claims 1 to 7, wherein the cancer is a solid carcinoma.

9. A system for predicting cancer prognosis, the system comprising:

a backbone, the backbone comprising a first computing layer, a second computing layer and a third computing layer, wherein the first computing layer used to identify a radiomics with a cancer marker, the second computing layer used to identify a pathological eigenvalue of the cancer marker, the third computing layer integrates the first computing layer and the second computing layer to establish an identification model, wherein the radiomics is the reference radiomics or the test radiomics, the pathological eigenvalue is the reference pathological eigenvalue or the test pathological eigenvalue;
a fourth computing layer configured for training the backbone to identify the radiomics with changes in cancer prognosis according to the identification model;
a fifth computing layer configured for training the backbone to identify the pathological eigenvalue with changes in cancer prognosis according to the identification model; and
a fully-connected computing layer with a prognostic index model configured for integrating the data output by the backbone, the fourth computing layer and the fifth computing layer to calculate a prognostic index, wherein the pathological eigenvalue is based on pathological features of a patient, the pathological features comprising genomic features, gene expression, test values or a combination of two or more thereof, and the prognostic index model has a mathematical formula as follows:

$$prognostic\ index = \frac{f_2(U_2, X_2)\pi_2}{f_1(U_1, X_1)\pi_1}$$

, wherein $U_1$ is the reference pathological eigenvalue; $X_1$ is the reference radiomics; $U_2$ is the test pathological eigenvalue; $X_2$ is the test radiomics; when the prognostic index is greater than or equal to 1, it is evaluated that the risk of prognostic change of the test patient is higher than or equal to that of the reference patient; when the prognostic index is less than 1, it is evaluated that the risk of prognostic change of the test patient is lower than that of the reference patient.

10. The system according to claim 9, further comprising:
a sixth computing layer used to capture the radiomics, and the step of the capturing the radiomics comprising:

capturing a lesion image, capturing a plurality of image feature variable values from the lesion image, outputting an image format data, and then normalizing the image format data with a dimensionality reduction matrix to obtain the radiomics, wherein the lesion image is a lesion image of a reference patient or a lesion image of a test patient, the radiomics is a reference radiomics or a test radiomics, so that the first computing layer can identify the reference radiomics or the fourth computing layer can identify the test radiomics.

11. The system according to any of claims 9 or 10, wherein the gene expression comprises RNA sequencing expression or protein expression.

12. The system according to any to claims 9 to 11, wherein the genomic features comprise gene copy number, gene mutant site, and single nucleotide polymorphisms ,SNPs.

13. The system according to any of claims 11 or 12, wherein when the gene expression is an RNA sequencing, the method further comprises:
normalizing a gene reading with a following formula to obtain the RNA sequencing expression:

$$\text{RNA sequencing expression} = \frac{\text{gene reading}}{\text{whole genome reading x gene base length}}$$

, wherein the gene reading is the RNA sequence reading of the reference gene or the test gene; the whole genome reading is the RNA sequence reading of the whole genome of the reference patient or the test patient; the gene base length is the base length of the reference gene or the test gene.

14. The system according to any of claims 9 to 13, wherein the reference image is one of a CT image, an fMRI image, an X-ray image, an ultrasound image or a pathological tomography image; the test image is one of a CT image, an fMRI image, an X-ray image, an ultrasound image or a pathological tomography image.

15. The system according to claim 9, wherein the cancer is a solid carcinoma.

101

capturing a reference
radiomics

102

obtaining a reference
pathological eigenvalue

103

capturing a test radiomics

obtaining a test pathological
eigenvalue

104

105

using a mathematical
formula to calculate a
prognostic index, and a risk
level of prognostic change of
the test patient is evaluated
according to the prognostic
index

FIG. 1

201

capturing a reference lesion image

202

capturing a plurality of image feature variable values from the reference lesion image and outputting a reference image format data

203

normalizing the reference image format data with a dimensionality reduction matrix to obtain a reference radiomics

FIG.2A

204

```
capturing a test lesion image
```

205

```
capturing a plurality of
image feature variable
values from the test lesion
image and outputting a test
image format data
```

206

```
normalizing the test image
format data with a
dimensionality reduction
matrix to obtain a test
radiomics
```

# FIG.2B

| a first computing layer | a second computing layer |

a fully-connected computing layer

| a third computing layer |

| a fourth computing layer | a fifth computing layer |

FIG.3A

a fully-connected computing layer

| a first computing layer | a second computing layer |

| a sixth computing layer | a third computing layer |

| a fourth computing layer | a fifth computing layer |

# FIG.3B

Image data     RNA-Seq dataset

21    71    6

FIG.4

FIG.5A

FIG.5B

FIG.6A

FIG.6B

FIG.6C

y

FIG.7A

FIG.7B

FIG.8

Europäisches Patentamt

European Patent Office

Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/003555 A1 (FERTE CHARLES [US] ET AL) 7 January 2021 (2021-01-07) * para. 1; para. 16; para. 21; para. 22, para. 100; Example 1; Example 2 * ----- | 1-15 | INV. G16B40/00 G16H50/30 |
| A | US 2020/365268 A1 (MICHUDA JACKSON [US] ET AL) 19 November 2020 (2020-11-19) * paragraphs [0014], [0015], [0031], [0129] * ----- | 1-15 | |

**TECHNICAL FIELDS SEARCHED (IPC)**

G16H
G16B

The present search report has been drawn up for all claims

| Place of search | Date of completion of the search | Examiner |
|---|---|---|
| Munich | 22 June 2023 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 3706

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2023

| Patent document cited in search report | | Publication date | Patent family member(s) | | Publication date |
|---|---|---|---|---|---|
| US 2021003555 | A1 | 07-01-2021 | AU | 2018298890 A1 | 06-02-2020 |
| | | | CA | 3069612 A1 | 17-01-2019 |
| | | | CN | 111094977 A | 01-05-2020 |
| | | | EP | 3652534 A1 | 20-05-2020 |
| | | | IL | 271996 A | 27-02-2020 |
| | | | JP | 2020526844 A | 31-08-2020 |
| | | | KR | 20200040754 A | 20-04-2020 |
| | | | US | 2021003555 A1 | 07-01-2021 |
| US 2020365268 | A1 | 19-11-2020 | AU | 2020274091 A1 | 09-12-2021 |
| | | | CA | 3140365 A1 | 19-11-2020 |
| | | | EP | 3970152 A1 | 23-03-2022 |
| | | | JP | 2022532897 A | 20-07-2022 |
| | | | US | 2020365268 A1 | 19-11-2020 |
| | | | US | 2023187070 A1 | 15-06-2023 |
| | | | WO | 2020232033 A1 | 19-11-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Journal of the American statistical association,* 2019, vol. 114 (527), 1305-1319
- *IEEE Access,* 2020, vol. 8, 22874-22883

- *IEEE Transactions on Radiation and Plasma Medical Sciences,* 2019, vol. 3 (2), 210-215